# EUROPEAN PATENT APPLICATION

(11) **EP 4 640 109 A1**
(43) Date of publication of application: **29.10.2025**
(21) Application number: 22969083.9
(22) Date of filing: 23.12.2022
(51) Int. Cl.: A45D 34/02, A61L 9/12

(54) **PERFUME BOTTLE AND SPRAYING SYSTEM**

(71) Applicant: Signature Parfumes, S.L., 41020 Sevilla (ES)
(72) Inventor: MEDINA RIVERO, Armando, 41020 Sevilla (ES)
(74) Representative: Ungria López, Javier
(86) International application number: PCT/ES2022/070835
(87) International publication number: WO 2024/133982

(57) **Abstract**

The present invention relates to a perfume bottle comprising a head (1) with an atomising nozzle (2) oriented upwards at a specific angle with respect to the longitudinal axis of the bottle and a regulating mechanism by means of which it can deliver sprays at partial percentages. It can have a manual application or form a system with the bottle located at a base (19) to have an electromechanical application from the base (19), both of the individual bottle and of a set of bottles located at the same base (19) in a circular configuration, so as to obtain a specific mixture of perfumes applied to a user or in a refill bottle (21).

## Description

### OBJECT OF THE INVENTION

The present invention relates to a perfume bottle, or a bottle containing hydroalcoholic compositions, incorporating a sprayer outlet oriented upwards at a specific angle with respect to the longitudinal axis of the bottle and a regulating mechanism by means of which it can deliver sprays at partial percentages. It can be manually actuated or form a system, with the bottle located at a base to be electromechanically actuated through the base, both the individual bottle and a set of bottles located in a facing or circular configuration at the same, so as to obtain a specific mixture of perfumes applied to a user or to an empty bottle for refilling.

It is particularly applicable in the field of the perfumery industry, particularly in perfume bottles with a sprayer and automatic dispensing devices.

### BACKGROUND OF THE INVENTION

There is a common practice carried out by both fragrance manufacturers and users, called "layering", which consists of mixing commercial perfumes to create a personalised fragrance. One way to carry out layering consists of the creation and marketing, by manufacturers, of fragrances or essences intended to be mixed together. Another way consists of combining several perfumes from different families that may or may not be from the same manufacturer, to create a new fragrance. The latter can also be a proposal from manufacturers, creating mixtures of the products they sell, although it is more an initiative of users, either to personalise a perfume to be unique, or for the purpose of making it less expensive by combining several perfumes of different prices.

The proposal of manufacturers that offer perfumes for layering consists of having two or more perfumes and, by means of complete sprays on a part of the body, achieving the desired mixture. This technique is limited to mixtures where one spray is the only unit of measurement that can be taken, so it would be very limited. An exemplary embodiment with three perfumes is described below:
- 1 spray of perfume 1 + 1 spray of perfume 2 + 1 spray of perfume 3; a perfume would be obtained with proportions of 33.33% / 33.33% / 33.33%, respectively, with a total result of 3 sprays.
- 2 sprays of perfume 1 + 1 spray of perfume 2 + 1 spray of perfume 3; a perfume would be obtained with proportions of 50% / 25% / 25%, respectively, with a total result of 4 sprays.
- 3 sprays of perfume 1 + 1 spray of perfume 2 + 1 spray of perfume 3; a perfume would be obtained with proportions of 60% / 20% / 20%, respectively, with a total result of 5 sprays.

As can be seen, to lower the proportion of one of the perfumes it would be necessary to raise the proportion of the others quite a bit, so in addition to using more product, there comes a point where the mixture would be nonviable due to the amount of perfume used, causing the perfume to be applied in the form of a stream on the part of the body to which it is applied instead of being sprayed.

Document EP3880259A1 consists of a device that incorporates a casing and a rotatable carousel, as a revolver, for housing several cartridges with perfume that are to be positioned sequentially so that, by means of an actuator, a specific amount of perfume is extracted from their inside to deposit it in a bottle positioned therebelow. This receiving bottle is a conventional bottle from which the atomiser has been removed, so it is filled with the desired proportion of the different perfumes.

The cartridge used would be the one described in document EP3849622, which consists of a perfume bottle with the customary elements of commercially available bottles, with the exception of the atomiser, which it lacks, and in its place there is a tube that drives the perfume in a liquid state downwards, all surrounded by a casing.

This invention has the drawback that the cartridges used cannot be used directly as unitary perfume bottles and free from the device, in addition to the fact that there cannot be any sprayed samples of the mixtures, but rather the bottle must be refilled in order to know the result of a specific mixture. This device could be included in machinery for making perfumes in a laboratory, unlike the one proposed, which has clear advantages for the user.

Document WO2021061056 discloses an electronically controlled perfume dispensing device that performs spraying by means of a mechanism located inside a casing. The device includes several bottles so that a user can select a suitable mixture in a personalised manner.

The technique used consists of transferring fluids by means of elements specific for that purpose, primarily a pump, solenoid valves and check valves, compressing the mixture of fluids on the same atomiser.

This invention has the drawback that it uses a unified fluids system, so that, when preparing a mixture, when a first fragrance is used, all the common and fixed elements of the device are impregnated with it. When using the next bottle to extract product from it, all the elements are already contaminated with the previous product. As a consequence, unwanted mixtures are obtained due to contamination from previous perfumes which substantially modify them.

To one of ordinary skill in the art, the composition of a perfume is known to also contain, in addition to the substances that provide the characteristic odours of the perfume itself, fixative elements or substances, which are intended to ensure the adherence and permanence of the perfume on the applied part, contaminating any part or element through which the perfume flows, and in particular non-removable elements such as those listed in the related patent.

The present invention solves these problems that were not overcome in the current state of the art and presents a perfume bottle that can be used for being applied to a user in small doses, and where a plurality of them can also be used in a device to apply a specific combination.

### DESCRIPTION OF THE INVENTION

The present invention refers to a perfume bottle that has a series of differentiating features with respect to bottles that are already commercially available.

The bottle is made up of a head, which includes an atomising nozzle through which the atomised perfume comes out, a container, as a housing where the perfume is located, and a body surrounding the container and through the inside of which the head slides to perform sprays. It should be noted that the container, together with the head, incorporates all the elements known in the prior art to enable spraying simply by pressing the head.

A first feature of the bottle of the invention consists of the atomising nozzle having an upward inclination at a specific angle, preferably between 40 and 50 degrees, with respect to the longitudinal axis of the bottle. Spraying is carried out at this angle so that the perfume is delivered slightly upwards instead of being at 90 degrees with respect to the longitudinal axis of the bottle and the perfume being delivered horizontally, as is known in the prior art.

A second feature of the bottle is that it incorporates a regulating mechanism through which sprays can be delivered at partial percentages. Regulation at values that are easy to understand by users can be considered, such as, for example, 25%, 50%, 75% and 100% of the spray. To achieve this regulation, the head of the bottle incorporates markings with the spray percentage to be delivered. The head is attached to the body of the bottle by means of a ramp mechanism or, alternatively, a toothed mechanism, which allows the spray not to be complete but only partial according to the amount determined. Thus, by rotating the head, the mechanism is placed in the selected position.

The invention also relates to a perfume bottle, and in particular its enclosure, which can incorporate two operating elements, for which it comprises two activating elements, the push button and an actuator.

A first activator is the push button on the head of the bottle, intended to be activated by the user in a conventional manner, by simply pressing, so that the liquid comes out of the atomising nozzle, as in any known perfume bottle. This activator is incorporated in the described bottle.

A second activator is an actuator, intended for use of the bottle as an electromechanical device. It consists of an operating projection located at the bottom of the bottle, connected to an internal mechanism. This actuator is intended for combined use with a motorised base from which the projection is pushed to perform sprays.

If the motorised base is used, the regulating mechanism to emit partial sprays is electronic, acting directly on the motor and in particular regulating its stroke.

When the motor of the base applies the impulse on the projection of the bottle, the bottle will tend to move in the direction of the impulse received. To avoid this movement of the bottle and to avoid the impulse being applied exclusively to the actuator, on the one hand, the bottle incorporates a recess in the lower zone of the periphery while, on the other hand, the base incorporates a retaining mechanism which, once the driving force is applied, it causes tabs to be fixed in the recess of the bottle, preventing the movement thereof.

This retaining mechanism can be made up of manually actuated tabs located at the base which, once the bottle is housed at the base, is activated by pressure to be housed in the recess and thus retain the bottle against the movement that transmits the force of the actuator that causes the spraying of the perfume. However, the retaining mechanism may also be electromechanical, as will be described later.

Furthermore, the base may be intended to house a single bottle or also a plurality of bottles.

By means of this actuator, the invention becomes an electromechanical device with all the electronic elements known in the art and not described, such as a power supply, motors, MCUs, sensors, Wi-Fi connection or Bluetooth and others, in which the spraying of the perfume can be performed by means of an electronic device, such as a mobile phone, computer or the like, through an application.

The regulating and operating mechanism is individual for each perfume bottle, allowing combinations to be made between them at different percentages. To achieve this, it is enough to operate the mechanism of several bottles at the same time, with a specific percentage and aiming it directly towards a point intended to receive the mixture.

Combining all of the above, the invention allows three embodiments as described below.

A first embodiment consists of an individual perfume bottle with the particularity that it allows regulation, on the bottle itself, of the desired spray percentage, to be applied directly on the skin of a user as in any commercial bottle. The application is as usual, i.e. pressing the head to perform the spray.

A second embodiment consists of the electromechanical application of the sprayer, for which the invention is configured as a system that incorporates a motorised base that activates a projection located in the bottle. This embodiment is focused on a single bottle, or on several bottles being used together. This last case, as it is more generic and incorporates the case of a single bottle, will be the one that is described.

In this system, the bottles are arranged circularly on a base, all with the sprayer pointing towards a projected point in space where the user will preferably place their wrist or any desired body part. The user regulates the spray ratio of each perfume bottle by means of a regulation mechanism which, unlike the manually regulated bottle, is not physical but rather electronic. The regulation of the spray percentage is preferably carried out through an application intended for this purpose, which sends orders to each motor, which is responsible for operating the actuator of the corresponding bottle to spray the mixture in a synchronised manner. However, it can also be carried out by means of a physical selector programmed to act on the motor. This technique allows perfume to be applied with various perfumes with a wide range and accuracy of proportions, as the regulation is electromechanical, and can regulate spraying by intervals of 1% or even less.

Given the possibility of regulating the bottles, this technique allows an infinite number of combinations by means of using several bottles. If extrapolated to the above examples, it can be seen that it allows mixtures to be made with a much smaller amount of perfume, avoiding coming out in the form of a stream onto the user's body, as mentioned above. With a specific regulation of a standard spray, the above example would be modified as shown below:
- 1 spray (reg. 25%) perfume 1 + 1 spray (reg. 25%) perfume 2 + 1 spray (reg. 25%) perfume 3, a perfume with proportions of 33.33% / 33.33% / 33.33%, respectively, would be obtained, with a total result of 0.75 sprays, compared to the conventional method, which would be 3 sprays.
- 1 spray (reg. 50%) perfume 1 + 1 spray (reg. 25%) perfume 2 + 1 spray (reg. 25%) perfume 3, a perfume with proportions of 50% / 25% / 25%, respectively, would be obtained, with a total result of 1 spray, compared to the conventional method, which would be 4 sprays.
- 1 spray (reg. 75%) perfume 1 + 1 spray (reg. 25%) perfume 2 + 1 spray (reg. 25%) perfume 3, a perfume with proportions of 60% / 20% / 20%, respectively, would be obtained, with a total result of 1.25 sprays, compared to the conventional method, which would be 5 sprays.

A third embodiment consists of incorporating into the invention a perfume refill bottle. On this occasion, as in the previous one, the perfume bottles are arranged circularly on the base, but with the intention of refilling a bottle placed in a gap located at the centre of the base. This is achieved by regulating the motor speed so that the spraying rate of all bottles drops to the point of becoming a stream. The refill bottle is filled by means of a piece located at the centre, fixed to the bottles, like a funnel with a central hole, which would collect all the perfumes delivered to direct them towards an empty bottle placed below the hole of the funnel. The funnel is configured with embossments on the periphery to couple the different bottles and can also incorporate a shield intended to be located in front of the outlet of the atomising nozzles of the perfume bottles, to prevent the delivered perfume from falling outside the funnel in case of oversized sprays.

It is important to highlight the motor regulation technique applied to the actuator, so that the perfume is released in a liquid state instead of in a spray, since the composition of perfumes contains a high percentage of alcohols that are highly volatile; otherwise, this substance vital for the conservation and consistency of the perfume would be lost.

In this way, as a summary, it can be indicated that the present invention relates to a perfume bottle comprising a head, an atomising nozzle located in the head, a container, which houses both the perfume and a supply conduit to the atomising nozzle and a mechanism to perform sprays, which may include a pump, and a body with a longitudinal axis, where both the container and the head are housed.

The atomising nozzle has an inclination with respect to the longitudinal axis of the body of the bottle and comprises a regulating mechanism that determines a percentage of a spray, where the inclination is between 40 and 50 degrees, and the regulating mechanism provides a plurality of percentages of a spray, according to its location in different positions.

The regulating mechanism can be a ramp mechanism which allows continuous regulation of the spray percentage to be delivered, or a toothed mechanism which allows regulation with various spray percentages to be delivered.

Furthermore, the invention also relates to a perfume spraying system in which bottles of the described type are used.

However, for the use of the bottles in this system, the bottle comprises a projection in contact with the container and a recess, respectively for lifting movements to carry out the spraying, and for retention to fix the bottle during this lifting movement.

This system comprises a base with a housing for a bottle, and it comprises an actuator fixed to the base and associated with the housing for the bottle. It also comprises a motor for applying the sprays.

The spraying system may also comprise a plurality of housings for bottles at the base, with an actuator associated with each of the housings.

Although a single motor could be used for using the system with a plurality of bottles, it is preferable to use a motor for each actuator, so that the partial sprays for each bottle can be regulated independently and directly, without the need to include additional mechanisms.

The motor used for spraying, regardless of whether it is to be used on a base with a single bottle or on a base with several bottles, is adjustable in stroke, so the piston will advance more or less, depending on the spray percentage that the user wishes to apply.

In addition, the user can also regulate the motor speed, selecting between a high speed to apply sprays and a low speed so that the sprays come out in the form of a stream.

The use of the motor at low speed to apply perfume in a stream requires the spraying system to also incorporate a central gap at the base for housing a refill bottle, and a collector, with a funnel configuration, intended to be fixed to the bottles, comprising a central hole, so that the hole is aligned with said central hole. In this way, by selecting the sprays with the defined percentage, each bottle will project a stream of perfume that will fall into the collector to end up in the central hole and go to the refill bottle located therebelow. This operation will be repeated as many times as necessary until the refill bottle is filled with the desired amount.

As for the configuration of the actuator, on the one hand, the lifting mechanism may comprise a toothed wheel, a rack meshing with the toothed wheel, a piston integral with the rack, and guides comprising respective through slots for controlling the retaining mechanism. On the other hand, the retaining mechanism comprises a pair of tabs, each with a pair of legs joined by a pin that is housed in the respective slot, through which it can move.

### DESCRIPTION OF THE FIGURES

To complete the description, and for the purpose of helping to make the features of the invention more readily understandable, this description is accompanied by a set of figures constituting an integral part of the same, which by way of illustration and not limitation represents the following:
Figure 1 represents a longitudinal section view of a bottle of the invention in a deactivated or rest position.
Figure 2 represents a perspective view of the base with the bottle lifting mechanism and its components.
Figure 3 represents a perspective view of a bottle of the invention located at a base in the deactivated position.
Figure 4 represents a perspective view of a bottle of the invention located at a base in the activated position.
Figure 5 represents a perspective view of the base with the lifting mechanism for lifting the bottle and its components in the deactivated position.
Figure 6 represents a perspective view of the base with the lifting mechanism for lifting the bottle and its components in the activated position.
Figure 7 represents a bottle with the head separated from the body to show a toothed attachment mechanism by means of which partial sprays are made.
Figure 8 represents a diagram of the different positions of the head with respect to the body of the bottle in dependence of the desired spray percentage and a ramp mechanism as an alternative to the toothed mechanism.
Figure 9 represents a base with five bottles before being placed at the base for electromechanical operation.
Figure 10 represents the base of Figure 9 with the bottles already assembled and with a collecting element assembled on the bottles for filling a refill bottle located in a hole at the centre of the base.

A list of the references used in the figures is provided below:
1. Head.
2. Atomising nozzle.
3. Container.
4. Body of the bottle.
5. Recess.
6. Projection.
7. Retaining mechanism.
8. Lifting mechanism.
9. Toothed wheel.
10. Rack.
11. Piston.
12. Guides.
13. Slot.
14. Guide legs.
15. Pin.
16. Tab.
17. Ramp mechanism
17a. Support.
17b. Ramp.
18. Toothed mechanism.
18a. First element.
18b. Second element.
19. Base.
20. Gap.
21. Refill bottle.
22. Collector.
23. Hole.
24. Housings.

### PREFERRED EMBODIMENT OF THE INVENTION

The present invention relates to a perfume bottle that incorporates a sprayer outlet oriented upwards at a specific angle with respect to the longitudinal axis of the bottle and a regulating mechanism by which it can deliver sprays at partial percentages, either manually or electromechanically by means of a motorised base (19), in which case it can also be applied to several bottles at the same time.

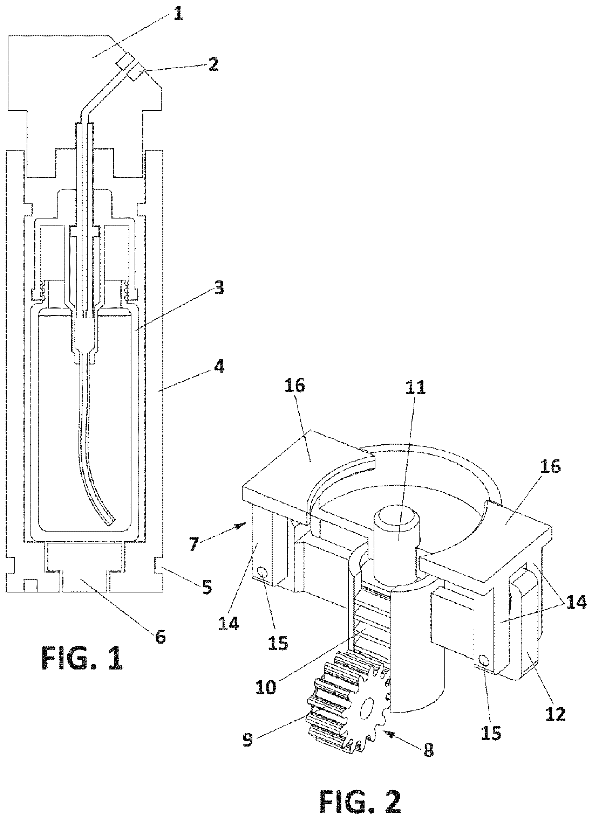

Figure 1 represents the bottle of the invention, where it can be seen that it is made up of a head (1), a container (3) that contains the perfume and the ejection mechanism, an atomising nozzle (2) through which the perfume exits to the outside, and a body (4) that surrounds the container (3) and houses the head (1).

Figure 2 represents an actuator responsible for carrying out the spraying electromechanically. To do this, it incorporates a retaining mechanism (7) and a lifting mechanism (8).

The lifting mechanism (8) comprises a toothed wheel (9), a rack (10), a piston (11) and guides (12).

The toothed wheel (9) is activated by the shaft of a motor, not represented in the figures.

The rack (10) meshes with the toothed wheel (9), comprises side walls and ends in a base.

The piston (11) is attached to the base of the rack (10) with the longitudinal axis in the direction of movement of the rack (10).

Each of the guides (12) incorporates a through slot (13) with a snaking shape.

The retaining mechanism (7) comprises a pair of tabs (16), each with a pair of legs (14) joined by a pin (15) that is housed in the respective slot (13), through which it can move.

The movement of the actuator, when it has a bottle housed, occurs as indicated below.

The actuator starts from a rest position, with the retaining mechanism (7) in the lower position. When the motor is activated, the toothed wheel (9) begins to rotate, causing the rack (10) to move.

This causes, on the one hand, the piston (11) to be lifted up, which pushes the projection (6) of the bottle to perform a spray.

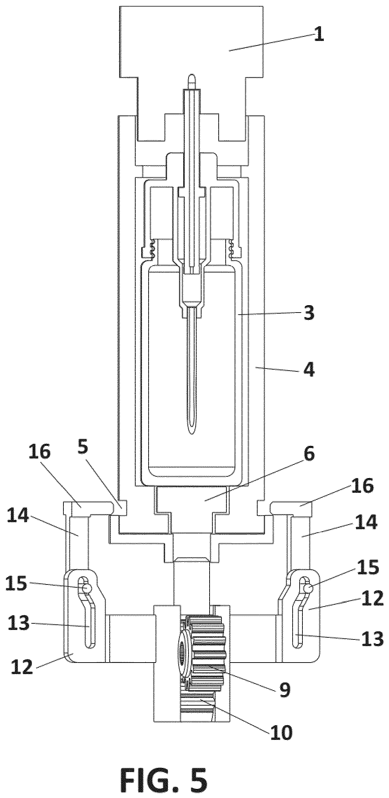

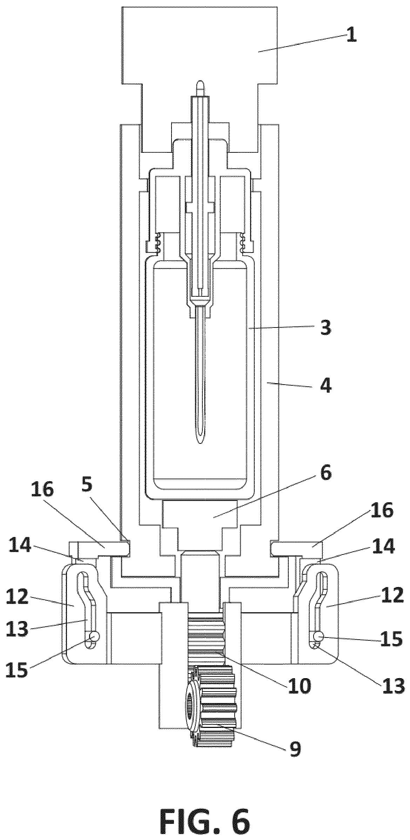

On the other hand, it also causes the guides (12) to be lifted up, which cause the pin (15) to move from the outermost position, in the top portion of the slot (13), as can be seen in Figure 5, to the innermost position, in the lower portion of the slot (13), as can be seen in Figure 6; Figures 5 and 6 representing a bottle located on the actuator, which has been sectioned to see how it is produced the movement of the retaining mechanism (7) to go, respectively, from the deactivated to the activated position.

With this movement, the tabs (16) go from a withdrawn position to a more closed position, being housed in the recess (5) incorporated in the body (4) of the bottle and preventing it from being able to move due to the force exerted by the piston (11).

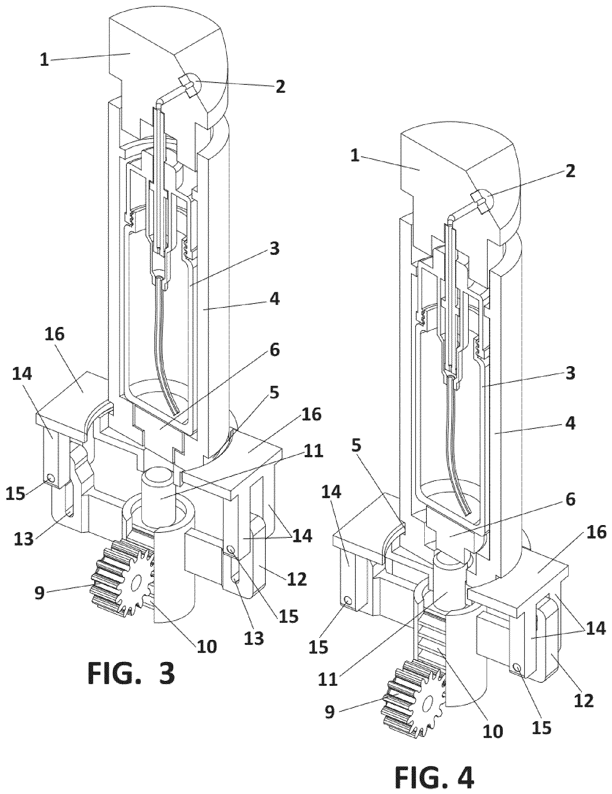

Figures 3 and 4 represent the bottle housed in an actuator in the deactivated or rest position, and in the activated position, once the delivery of a spray has started, respectively.

As mentioned, one object of the invention consists of the bottles being able to emit partial sprays, instead of the customary complete one, which can be of a large amount of product, especially in cases where the application of perfume by means of several bottles is sought.

The application of partial sprays manually can be carried out by means of a regulating mechanism located between the head (1) and the body (4) of the bottle. In that sense, the head (1) incorporates markings that define the spray percentage that is desired to be made and, by rotating it, the head (1) is positioned with respect to the body (4) of the bottle to emit the desired spray. Depending on the positions of these two elements, the movement will be more or less limited, causing a smaller or larger spray, respectively.

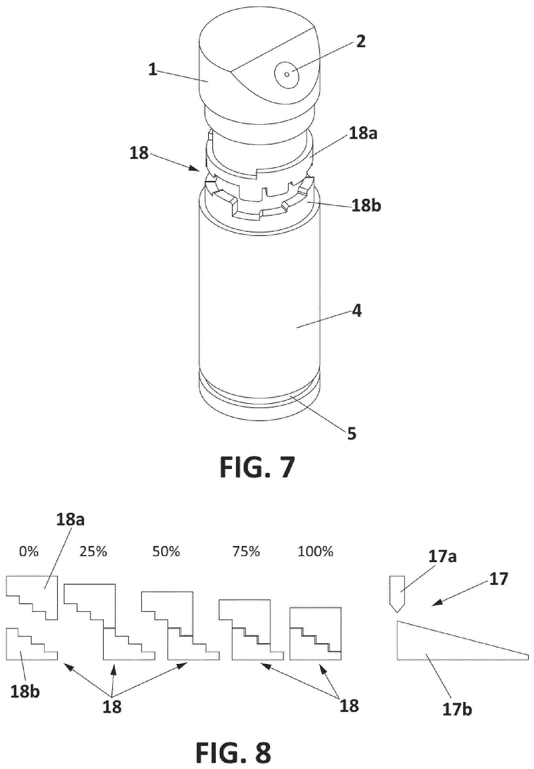

Figure 7 represents the bottle of the invention where the head (1) of the body (4) is shown in an exploded view to allow seeing the regulating mechanism responsible for performing sprays on a percentage basis. This figure shows that the regulatory mechanism consists of a toothed mechanism (18) formed by two elements connected to one another, a first element (18a) located in the head (1), in the lower zone, and a second element (18b) located in the body (4), in the upper zone.

Figure 8 represents the different positions that the regulatory mechanism can adopt for the use of a toothed mechanism (18), showing the different positions that the first element (18a) can adopt with respect to the second element (18b). It also represents a ramp mechanism (17) formed by a support (17a) and a ramp (17b), with the same principle as the toothed mechanism (18), although with a continuous regulation of the positions instead of a discrete, jerky regulation.

In this sense, said Figure 8 represents a planar simulation of the position of the two elements of the regulating mechanism in the two indicated embodiments. It can be seen how, in a rest position, the two elements (18a, 18b) of the toothed mechanism (18) are not in contact, and will only be in contact once a spray, of represented percentages of 25%, 50%, 75% and 100% of a spray, has been carried out, with the two parts of the regulating mechanism coming into contact in a certain position that will result in more or less movement of the body (4) with respect to the head (1) and will apply a larger or smaller spray, respectively. In the same way, the ramp mechanism (17) is in a rest position, where the support (17a) and the ramp (17b) are not in contact.

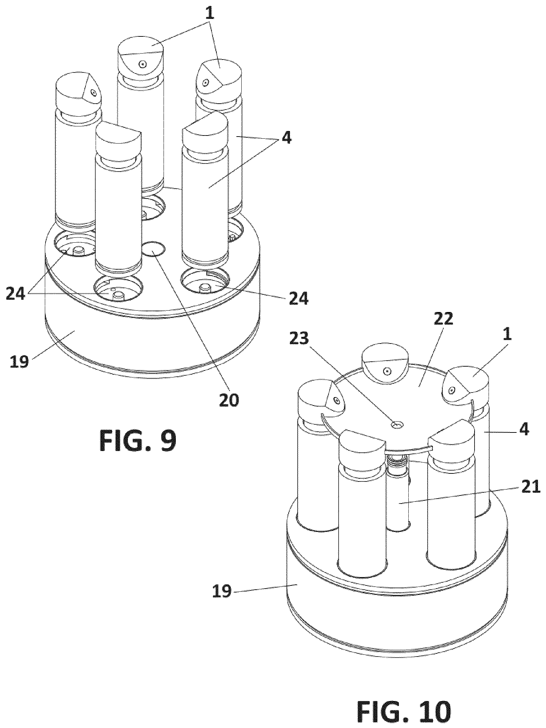

Figure 9 represents a base (19) with a plurality of housings (24) intended to house perfume bottles to carry out layering or simultaneous emission of several perfumes to obtain a different result. Each of the housings (24) of the bottles is associated with an actuator, and the base (19) can incorporate a motor for the activation of each of the actuators or for activating several, or incorporate a single motor for activating all the actuators. However, while not incorporating a motor for each actuator would avoid problems with synchronisation between sprays from the different bottles, it would have the added problem that an additional regulatory mechanism would have to be incorporated in each actuator, so that the movement of the motor acts differently on each actuator, acting more or less intensely, in dependence of the selected spray percentage.

Figure 10 represents the base (19) of Figure 9 with the bottles already located in the housings (24), also including a refill bottle (21) housed in a central gap (20) defined at the base (19), and a collector (22) fixed to the bottles below the atomising nozzles (2).

This configuration is for an embodiment in which the spraying rate is very small so that, instead of making sprays, the bottles emit liquid streams through the atomising nozzles (2) that fall onto the collector (22) and reach the refill bottle (21) through the central hole (23). Therefore, the motor speed must be adjustable by the user. To do this, the system can incorporate a push button which the user can access to choose between a spray position and a stream position. Alternatively, the push button can be electronic, so that the position is selected from an electronic application in a control device such as a mobile phone, a computer, a tablet, or the like.

The collector can incorporate screens, not represented, intended so that, in the event that the release of liquid perfume is more powerful than expected, the liquid will hit said screens and fall into the collector (22) instead of coming out of it.

Lastly, it must be taken into account that the present invention must not be limited by the embodiment described herein. Other configurations may be carried out by those skilled in the art based on the present description. Accordingly, the scope of the invention is defined by the following claims.

## Claims

1. A perfume bottle comprising a head (1), an atomising nozzle (2) located in the head (1), a container (3), which houses both the perfume and a supply conduit to the atomising nozzle (2) and a mechanism to perform sprays, and a body (4), with a longitudinal axis, where both the container (3) and the head (1) are housed, the bottle being **characterised in that**:
- the atomising nozzle (2) has an inclination with respect to the longitudinal axis of the body (4) of the bottle, and
- it comprises a regulating mechanism that determines a percentage of a spray,
where
- the inclination is between 40 and 50 degrees, and
- the regulating mechanism provides a plurality of percentages of a spray, according to the position in which it is located.

2. The perfume bottle according to claim 1, **characterised in that** the regulating mechanism is a ramp mechanism (17) which allows continuous regulation of the spray percentage to be delivered.

3. The perfume bottle according to claim 1, **characterised in that** the regulating mechanism is a toothed mechanism (18) which allows discrete regulation, with several spray percentages to be delivered.

4. A perfume spraying system in bottles according to the preceding claims, **characterised in that**:
- the bottle comprises a projection (6) in contact with the container (3) and a recess (5),
- it comprises a base (19) with a housing (24) for a bottle, and
- it comprises an actuator fixed to the base (19) and associated with the housing (24) of the bottle and a motor for applying the sprays,
where
- the actuator comprises, in turn,
- a lifting mechanism (8), connected to the motor, responsible for acting on the projection (6) and carrying out a spray; and
- a retaining mechanism (7), attached to the lifting mechanism (8), intended to fix the base (19) to the bottle through the recess (5) when spraying occurs.

5. The spraying system according to claim 4, **characterised in that**:
- the base (19) comprises a plurality of housings (24) for bottles,
- the motor speed is adjustable, and
- it comprises an actuator associated with each of the housings (24).

6. The spraying system according to claim 5, **characterised in that** it comprises:
- a central gap (20) for housing a filling bottle (21), and
- a collector (22), with a funnel configuration, intended to be fixed to the bottles, comprising a central hole (23), so that the hole (23) is aligned with the gap (20).

7. The spraying system according to claim 4, **characterised in that**:
- the lifting mechanism (8) comprises a toothed wheel (9), a rack (10) meshing with the toothed wheel (9), a piston (11) integral with the rack (10), and guides (12) comprising respective through slots (13) for controlling the retaining mechanism (7), and
- the retaining mechanism (7) comprises a pair of tabs (16), each with a pair of legs (14) joined by a pin (15) that is housed in the respective slot (13), through which it can move.
